# EUROPÄISCHE PATENTSCHRIFT

(11) **EP 1 495 723 B1**
(45) Veröffentlichungstag und Bekanntmachung des Hinweises auf die Patenterteilung: **28.06.2006**
(21) Anmeldenummer: 03015499.1
(22) Anmeldetag: 09.07.2003
(51) Int. Cl.: A61B 17/02

(54) **Spreizer für chirurgische Eingriffe**
Surgical tissue retractor
Ecarteur chirurgical

(43) Veröffentlichungstag der Anmeldung: 12.01.2005
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll

(56) Entgegenhaltungen:
- EP-A- 1 053 717
- WO-A-03/017847
- US-A- 569 839
- US-A- 5 529 571
- US-A- 6 042 540

## Beschreibung

Die Erfindung betrifft einen Spreizer für chirurgische Eingriffe insbesondere Subskapularis-Spreizer mit zwei Branchen, die an ihren einen Enden mit je einem Griff und an ihren anderen Enden mit einem Krallenelement mit einem sich im wesentlichen quer zur Branche erstreckenden Zinken versehen und zwischen den Enden scharnierartig verbunden sind, und mit einer Arretierungseinrichtung, wobei jedes Krallenelement mehr als einen Zinken aufweist und mit Hilfe eines Scharniers an der Branche befestigt ist, dessen Schwenkachse im wesentlichen quer zur Branche und im wesentlichen in Spreizrichtung ausgerichtet ist.

Mit den entsprechenden Fortschritten auf diesem Gebiet und wachsender Erfahrung werden Möglichkeiten, aber auch Grenzen endoskopisch kontrollierter Eingriffe an der Schulter deutlich. Außerhalb dieser Grenzen behalten offene Eingriffe ihre Bedeutung, und hier werden andererseits Bestrebungen zur Minimalisierung deutlich: sogenannte mini-open Technik z. B. beim Verschluß von Rotatorendefekten.

Insbesondere bei professionellen Athleten, bei denen die Schulter stark beansprucht wird (z. B. Baseball-pitcher) sind prominente Behandler immer schon bestrebt gewesen, die Trainings- und Funktionsfähigkeit möglichst schnell wiederherzustellen. Dazu gehört es, außer minimalen Zugängen keine Strukturen zu durchtrennen, die bei der sportlichen Tätigkeit stark belastet werden und vor erneuter Belastung erst wieder zusammenheilen müssen.

Beim klassischen Zugang zur Schultergelenkkapsel von vorn wird der M. subscapularis am Muskel-Sehenübergang quer durchtrennt. Nach dem entsprechenden Eingriff am Kapsel-Bandapparat muß der M. subscapularis wieder vernäht werden. Dabei dauert es natürlich längere Zeit bis diese quer zu den Muskelfasern verlaufende Nahtstelle verheilt und wieder belastbar ist. Der Zugang zur Kapsel ist aber auch durch Erzeugung eines Spalts im Muskel in Faserrichtung möglich, so daß keine Fasern durchtrennt werden müssen, was eine schnellere Heilung ermöglicht.

Die Präparation zur Freilegung der Kapsel ist dann aufwendiger. Dies wird dadurch kompensiert, daß statt der quer verlaufenden Naht nur der Längsspalt adaptiert zu werden braucht. Die Länge des Muskels und seine Belastbarkeit werden dabei unverändert erhalten. Entsprechende Untersuchungen haben im Vergleich zur klassischen Querdurchtrennung, wie man dies erwarten würde, deutliche Verteile in der Rehabilitation gezeigt.

Bisher gab es für das Spreizen und Offenhalten des Spaltes in dem Muskel kein geeignetes Instrument. Der M. subscapularis liegt auf dem Schulterblatt und verläuft schräg von außen oben nach innen unten. Ein modifizierter Gelpi-Spreizer von F. Jobe, der die Längsspaltung bei seinen Baseball-Pitchern benutzt hat, der von vorn eingesetzt wird, greift immer schräg zu den Muskelfasern an. Dieser Spreizer weist zwei scharnierartig verbundene Branchen auf, die an ihren einen Enden mit einem Griff und an dem anderen Ende mit einer Zinke versehen sind, die im wesentlichen senkrecht von dem Ende der Branche absteht. Diese Zinken greifen an den Muskelfasern an. Der Druck von einem Zinken beim Instrument von Jobe quetscht die Muskelfasern und reist wegen des schrägen Ansatzes auch gelegentlich aus. Senkrecht zu den Muskelfasern kann kein gerades Instrument angreifen, da nach medial der Brustkorb im Weg ist.

Die Notwendigkeit für einen verbesserten Spreizer ergibt sich also aus dem schrägen Verlauf des M. suscapsularis und der Erfahrung, daß ein Zinken, der im wesentlichen senkrecht zum Ende der Branche steht, nur schräg am Muskel angreifen kann. Dies führt zur Schädigungen der Muskulatur.

Bekannt sind Spreizer aus EP 1 053 717 A1, US 6,042,540 und WO 03/017847 A1. Diese sind jedoch dazu ausgelegt, in einer speziellen Ausrichtung an das Operationsobjekt herangeführt zu werden.

Die Aufgabe der Erfindung besteht in der Schaffung eines verbesserten Spreizers der Eingangs genannten Art, mit der der Spalt besser und sicherer ohne Beschädigung der Muskelfasern geöffnet und offengehalten werden kann.

Die erfindungsgemäße Lösung besteht darin, daß die Krallenelemente auf beide Seiten einer Ebene schwenkbar sind, in der die Endbereiche der Branchen liegen, an denen die Krallenelemente befestigt sind, und dass die Branchen aus einer senkrecht zur Achse des Branchenscharniers liegenden Ebene heraus gekrümmt sind. Dadurch ist es möglich, den Spreizer rechts und links jeweils von außen (vom Arm her) oder von innen (vom Brustkorb her) einzusetzen.

Aufgrund der scharnierartigen Befestigung kann sich das Krallenelement gerade an die Muskelfasern anlegen. Dabei können die mehr als eine Zinke nur aufgrund der Scharnierbefestigung gleichzeitig an den Muskelfasern angreifen. Die Krallenelemente können mit ihren Zinken in einem Winkel zu den Branchen des Spreizers aufgrund der Scharnierverbindung angeordnet werden und senkrecht zu den Muskelfasern angreifen. Der Druck auf die Muskelfasern wird auf die mehreren Zinken verteilt und wirkt quer zu den Muskelfasern. Das ist sicherer und schonender als bei einem Gelpi-Spreizer, der an jeder Branche nur einen Zinken aufweist. Das erfindungsgemäße Instrument kann je nach den Erfordernissen von medial und von lateral her eingesetzt werden.

Vorteilhafterweise weist jedes Krallenelement zwei Zinken auf, obwohl auch mehr als zwei Zinken pro Krallenelement vorgesehen sein könnten.

Die vorderen Endbereiche der Zinken sind zweckmäßigerweise im wesentlichen parallel zur Schwenkachse ihres Krallenelements.

Vorteilhafterweise sind die Branchen gekrümmt, und zwar in einer Ebene, die senkrecht ist zur Achse des Scharniers, mit dem die Branchen miteinander verbunden sind.

Die Erfindung wird im folgenden anhand einer vorteilhaften Ausführungsform unter Bezugnahme auf die beigefügten Zeichnungen beschrieben. Es zeigen:

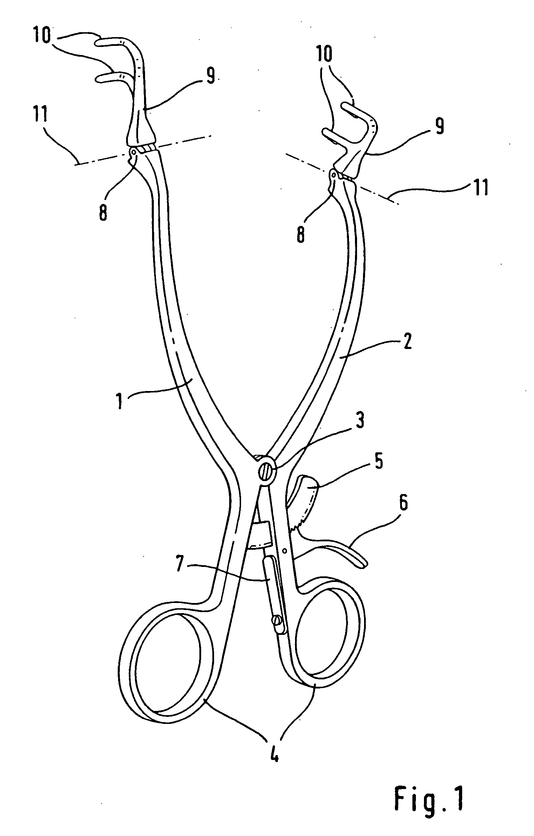

- Figur 1: eine perspektivische Ansicht eines erfindungsgemäßen Spreizers; und

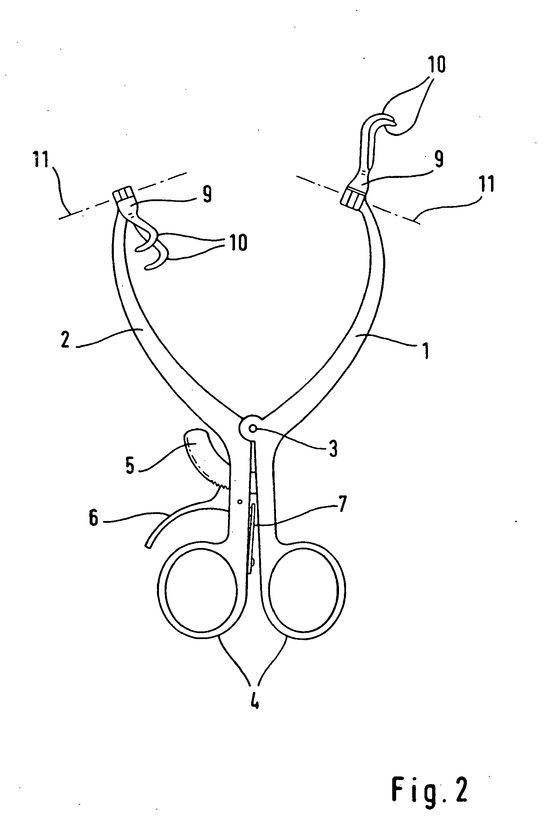

- Figur 2: eine weitere perspektivische Ansicht in einer etwas anderen Stellung.

Der erfindungsgemäße Spreizer weist zwei Branchen 1, 2 auf, die bei 3 scharnierartig verbunden sind. An einem Ende sind die Branchen 2 mit Griffen 4 versehen. Zwischen den Griffen 4 und der scharnierartigen Verbindung 3 befindet sich eine Sperreinrichtung. An der Branche 1 ist dazu ein gekrümmtes gezahntes Element 5 befestigt, in das eine Klinke 6 eingreifen kann, die an der Branche 2 befestigt ist. Diese Klinke 6 wird durch eine Feder 7 in Richtung auf das gezahnte Element 5 gedrückt. An den Enden der Branchen 1 bis 2, die von den Griffen 4 entfernt sind, sind mit Scharnieren 8 Krallenelemente 9 befestigt, die jeweils zwei Zinken 10 aufweisen. Die Achsen 11 dieser Scharniere 8 erstrecken sich dabei im wesentlichen quer zur Erstreckung der Branchen 1, 2 im Endbereich bei den Scharnieren 8 und liegen im wesentlichen in einer Ebene. Die Krallenelemente 9 mit den Zinken 2 können dabei auf beide Seite der Zeichnungsebenen verschwenkt werden, was die vielseitige Verwendung des Spreizers ermöglicht.

Werden die Griffe 4 zusammengedrückt, so werden die Krallenelemente 9 auseinandergedrückt und bewirken die Spreizung. Beim Zusammendrücken wird entweder die Klinke 6 gelöst, um die Spreizbewegung zu ermöglichen, oder aber es wird eine sägezahnförmige Verzahnung am Element 5 vorgesehen, die diese Spreizbewegung, aber nicht die umgekehrte Bewegung erlaubt. Die Spreizwirkung kann nach Beendigung des Eingriffs an der Schultergelenkkapsel durch Betätigung der Klinke 6 beendet werden.

## Patentansprüche

1. Spreizer für chirurgische Eingriffe, insbesondere Subskapularis-Spreizer, mit zwei Branchen (1, 2), die an ihren einen Enden mit je einem Griff (4) und an ihren anderen Enden mit je einem Krallenelement (9) versehen sind und die zwischen den Enden scharnierartig über ein Branchenscharnier (3) verbunden sind, wobei die Krallenelemente (9) mit mehr als einem sich im wesentlichen quer zur Branche (1, 2) erstreckenden Zinken (10) versehen sind und mit Hilfe eines Scharniers (8) an der Branche (1, 2) befestigt sind, dessen Schwenkachse (11) im wesentlichen quer zur Branche (1 ,2) und im wesentlichen in Spreizrichtung ausgerichtet ist, so daß die Krallenelemente (9) auf beide Seiten einer von der Achse des Krallenscharniers und den Endbereichen der Branchen (1, 2) gebildeten Ebene schwenkbar sind und mit einer Arretierungseinrichtung (5, 6, 7), **dadurch gekennzeichnet, daß** die Branchen (1, 2) aus einer senkrecht zur Achse des Branchenscharniers (3) liegenden Ebene heraus gekrümmt sind.

2. Spreizer nach Anspruch 1, **dadurch gekennzeichnet, daß** jedes Krallenelement (9) zwei Zinken (10) aufweist.

3. Spreizer nach Anspruch 1 oder 2, **dadurch gekennzeichnet, daß** die vorderen Endbereiche der Zinken (10) im wesentlichen parallel zur Schwenkachse (8) ihrer Krallenelemente sind.

4. Spreizer nach einem der Ansprüche 1 bis 3, **dadurch gekennzeichnet, dass** die Branchen (1, 2) innerhalb einer Ebene senkrecht zur Achse des Branchenschaniers (3) gekrümmt sind.

## Claims

1. Retractor for surgical interventions, in particular a subscapular retractor, with two branches (1, 2) which are each provided with a grip (4) at one end and with a claw element (9) at the other end and which between the ends are connected in an articulated manner via a branch hinge (3), the claw elements (9) being provided with more than one tine (10) that extends substantially transverse to the branch (1, 2) and being secured on the branch (1, 2) with the aid of a hinge (8) whose pivot axis (11) is oriented substantially transverse to the branch (1, 2) and substantially in the spreading direction, such that the claw elements (9) are pivotable to both sides of a plane formed by the axis of the claw hinge and the end areas of the branches (1, 2), and with a locking device (5, 6, 7), **characterized in that** the branches (1, 2) curve out from a plane lying perpendicular to the axis of the branch hinge (3).

2. Retractor according to Claim 1, **characterized in that** each claw element (9) has two tines (10).

3. Retractor according to Claim 1 or 2, **characterized in that** the front end areas of the tines (10) are substantially parallel to the pivot axis (11) of their claw elements.

4. Retractor according to one of Claims 1 to 3, **characterized in that** the branches (1, 2) are curved inside a plane perpendicular to the axis of the branch hinge (3).

## Revendications

1. Ecarteur pour interventions chirurgicales, notamment écarteur sous-scapulaire, à deux branches (1, 2) qui sont pourvues à une extrémité chacune d'une poignée (4) et à leur autre extrémité chacune d'un élément à griffes (9) et sont reliées entre les extrémités à la manière d'une charnière par une charnière de branches (3), les éléments à griffes (9) étant pourvus de plus d'une griffe (10) s'étendant sensiblement transversalement par rapport à la branche (1, 2) et étant fixés à la branche (1, 2) à l'aide d'une charnière (8) dont l'axe de pivotement (11) est orienté sensiblement transversalement par rapport à la branche (1, 2) et sensiblement dans le sens d'écartement, de sorte que les éléments à griffes (9) peuvent pivoter des deux côtés d'un plan constitué par l'axe de la charnière de griffes et les zones terminales des branches (1, 2), et comportant un dispositif de blocage (5, 6, 7), **caractérisé en ce que** les branches (1, 2) sont recourbées depuis un plan placé perpendiculairement à l'axe de la charnière de branches (3).

2. Ecarteur selon la revendication 1, **caractérisé en ce que** chaque élément à griffes (9) présente deux griffes (10).

3. Ecarteur selon la revendication 1 ou 2, **caractérisé en ce que** les zones terminales avant des griffes (10) sont sensiblement parallèles à l'axe de pivotement (11) de leurs éléments à griffes.

4. Ecarteur selon une des revendications 1 à 3, **caractérisé en ce que** les branches (1, 2) sont recourbées dans un plan perpendiculaire à l'axe de la charnière de branches (3).
